# NEUE EUROPÄISCHE PATENTSCHRIFT

(11) **EP 2 161 011 B2**
(45) Veröffentlichungstag und Bekanntmachung des Hinweises auf die Entscheidung über den Einspruch: **20.05.2015**
(45) Hinweis auf die Patenterteilung: 20.06.2012
(21) Anmeldenummer: 09169101.4
(22) Anmeldetag: 01.09.2009
(51) Int. Cl.: A61F 13/00

(54) **Wundschutz**
Wound protection
Protection de plaies

(30) Priorität: 03.09.2008 EP 08105217
(43) Veröffentlichungstag der Anmeldung: 10.03.2010
(73) Patentinhaber: Pistor, Gert, 14195 Berlin (DE)
(72) Erfinder: Pistor, Gert, 14195 Berlin (DE)
(74) Vertreter: Jäger, Gerhard Fred

(56) Entgegenhaltungen:
- DE-U1-202005 019 670
- FR-A- 1 104 098
- FR-A1- 2 583 636
- FR-A1- 2 661 821
- US-A- 5 495 856
- US-A- 6 013 097
- US-A1- 2005 090 791
- US-A1- 2005 222 528
- US-B1- 6 458 109

## Beschreibung

Die Erfindung betrifft eine Wundabdeckung mit einer oder mehreren Kappen und einem Träger, insbesondere eine Penis-Wundabdeckung, und deren Verwendung.

Verbandsprobleme treten oft bei Operationswunden auf, insbesondere bei Penisoperationen.

Von besonderer Bedeutung ist die mit dem medizinischen Fortschritt einhergehende rasante Zunahme der ambulanten Operationen und der nachfolgende Transport der frisch Operierten in Privatwagen, die mit den handelsüblichen Angurtsystemen ausgestattet sind. Die für Kinder empfohlenen und sicheren Rückhaltesysteme belasten konstruktionsbedingt die bei den Kindern häufigsten Operationsgebiete am Penis sowie der Leisten- und Hodenregion. Zumeist steht der behandelnde Arzt hier in einem Entscheidungszwiespalt. Einerseits ist das korrekte Anlegen der Sicherungsgurte für einen sicheren Transport und die Transportsicherheit gefordert und behördlich angeordnet, andererseits sind der Gurtdruck und alle Scherbewegungen auf die Wunde unter einem straff angelegten Gurt problematisch für die Wundstabilität.

Ähnliche Probleme bestehen bei dem Transport von operierten Kindern im Kinderwagen und in Tragetaschen, insbesondere Rücken- und Bauchtragetaschen.

Ein trägerloser Wundschutzdeckel wird in der DE 321687 A , ein klebbarer Deckel in der FR 701472 A, eine aufklebbare gewölbte Membran in der DE 215015 A und eine auf die Haut aufklebbare Unterdruckkammer in der US 3486504 A beschrieben.

Eine kontaktfreie Wundabdeckung ist von Pflastermaterialien bekannt, die beispielsweise in der DE 69535208 T2, CH 269938 A, US 4212296 A, US 3486504 A, US 2443140 A und DE 1767134 U beschrieben werden. Nachteilig sind die klebende Fixierung auf der Haut und die mangelnde Stabilität.

Ein hüllenartiges Suspensorium mit Haltebändern ohne mechanische Schutzfunktion ist aus der DE 8716904 U1 und eine Tasche mit adhäsiven Haltebändem zur Aufnahme der männlichen Genitalien ist aus der US 4378010 A bekannt.

Pflasterartige oder adhäsive Wundabdeckungen zur direkten Befestigung auf der Haut werden ferner in der FR 2583636 A1, FR 2661821 A1, FR 1104098 A, US 5495856 A1 und US 6013097 A beschrieben.

Die US 2005/090791 A1 behandelt einen stoßabsorbierenden Hygieneartikel wie eine Windel, wo eine Schutzschale in der Windel zwischen Innen- und Außenhülle oder unter der Windel am Körper angeordnet wird.

Der Erfindung liegt die Aufgabe zugrunde, ein alternatives Verbandmittel bereit zu stellen.

Gelöst wurde die Aufgabe durch eine Wundabdeckung mit den in Anspruch 1 beschriebenen Merkmalen.

Die Wundabdeckung umfasst mindestens eine Kappe. Der Begriff Kappe steht im Folgenden für ein gewölbtes oder gebogenes Abdeckmittel. Der Begriff Kappe schliesst also Kappen, kappenartige Gebilde und Bügel ein.

Der Begriff Wundabdeckung umfasst eine Abdeckung in der Art einer Überdachung von Arealen der Haut für alle Arten einer medizinischen, therapeutischen, prophylaktischen, kosmetischen oder schützenden Verwendung am Körper eines Menschen oder Tieres. Solche abzudeckenden Hautareale sind beispielsweise neben Wunden oder Verwundungen auch wunde oder irritierte Hautareale, befallene, kranke oder krankhaft veränderte Hautflächen (z.B. Pilzbefall, Schuppenflechte oder neurodermitische Herde) oder zu schützende Hautareale, die in der Regel empfindliche Stellen der Haut darstellen. Von dem Begriff Wunde werden im Folgenden solche Stellen der Haut mit umfasst.

Eine kontaktfreie Abdeckung eines Hautareals bedeutet, dass jeglicher Kontakt der Haut in dem Hautareal mit der Kappe, dem Träger oder einem sonstigen Material durch die Abdeckung vermieden werden. Eine kontaktfreie Wundabdeckung verhindert den Kontakt der Wunde mit dem Verbundmaterial oder Fremdmaterial.

Im einfachsten Fall ist die Kappe eine gewölbte oder gebogene Fläche. Eine gebogene Fläche ist z.B. ein Bügel, der in der Breite variiert werden kann und insbesondere so breit wie die Wunde sein kann. Vorteilhaft ist ein kreuzförmiger Bügel (gewölbtes Kreuz) oder zwei oder mehrere gekreuzte Bügel. Die Bügel sind z.B. aus Kunststoff. Am Bügel sind z.B. ein, zwei, drei oder mehrere Befestigungsmittel oder Befestigungselemente angebracht, womit der Bügel mit dem Träger verbunden wird.

Vorteilhaft verfügt die Kappe über einen flachen Rand, der z.B. abgewinkelt ist und eine flache Auflage auf der Haut oder dem Träger bildet. Ist die Kappe auf einem Träger mit einer Öffnung im Bereich der Wunde angeordnet, so ist die Kappe in der Regel in der Trägerebene oder im Bereich der Trägerebene mit dem Träger verbunden. Dies hat bei abnehmbarer Kappe den Vorteil, dass die Wunde bzw. das abgedeckte Hautareal besser zugänglich ist. Bei entfernter Kappe ist die Wunde seitlich frei und ist nicht durch hoch stehende Trägerteile oder andere aufragende Teile umgeben.

Eine alternative Ausführungsform umfasst einen Träger ohne Öffnung im Bereich der Wunde und eine Kappe, die auf dem Träger angeordnet ist und durch geeignete Mittel (z.B. Befestigungsmittel) den Träger an der Innenseite oder Teilen der Innenseite der Kappe hält. Auf diese Weise wird der Träger im Bereich der Wunde in einem Abstand zur Wunde gehalten. Die Kappe ist vorzugsweise abnehmbar. Bevorzugtes Befestigungsmittel ist ein lösbares Befestigungsmittel, insbesondere Klettbandmaterial.

Die Kappe ist vorzugsweise einteilig. Einteiligkeit bedeutet, dass die Kappe aus einem Stück oder einem aus Einzelteilen, in der Regel nicht trennbar, zusammengesetzten Stück als Hauptbestandteil besteht. Das Ha uptbestandteil ist insbesondere ein schalenartig, korbförmig oder kuppelartig geformtes Teil. Das Hauptbestandteil kann weitere Bestandteile tragen, aufweisen oder enthalten, die aber nicht grundlegend zur Ausbildung einer Kappe, der Kappeneigenschaft oder einer Abdeckung beitragen. Die Kappe wird im Allgemeinen als Gesamtheit befestigt, angebracht, entfernt oder ausgetauscht.

Die Kappe besitzt in der Regel eine ausreichende Festigkeit, um nicht bei Einwirkung äußerer Kräfte zusammengedrückt zu werden. Diese Eigenschaft der Kappe wird als Formstabilität bezeichnet. Die Stabilität der Kappe kann je nach Anwendung variiert werden. Die Kappe ist vorzugsweise formstabil oder weitgehend formstabil, was durch geeignete Wahl des Materials oder durch eine Verstärkung, Versteifung (z.B. durch Rippen, Rillen, Bögen), Verstrebung oder Formgebung (z.B. Bögen, Wellen, Kanten) des Materials erreicht wird. Das Material ist z.B. ein einheitliches Material wie Kunststoff (thermoplastischer oder duroplastischer Kunststoff), Textil (z.B. Gewebe, Vlies, Filz), Keramik, Metall, Pappe oder eine Kombination von zwei oder mehreren Materialien, z.B. verbundenes Material (z.B. verklebtes, verschweißtes oder mechanisch verbundenes Material), ein Verbundmaterial (z.B. Faserverbundmaterial wie Epoxid/Glasfaser, Polyester/Carbonfaser usw.), Laminat (z.B. Kunststofffolien, Kunststofffolie/Papier oder Kunststofffolie/Pappe), ein Kunstharz getränktes Material (z.B. Kunstharz getränktes Textil, Pappe, Papier). Die Kappe besteht vorzugsweise ganz oder teilweise aus Kunststoff. Die Kappe ist z.B. ein Formteil aus einem thermoplastischen Kunststoff (z.B. extrudiertes, blasgeformtes oder tiefgezogenes Teil).

Im Bereich des an der Grundfläche der Kappe gelegenen Randes ist die Kappe vorteilhaft durchgehend (z.B. ringförmig), unterbrochen oder stellenweise mit einem weicheren oder weichen Material ausgestattet, z.B. als Einlage oder Auflage. Ein weicheres Material ist z.B. Schaumstoff, geschäumtes Material, elastischer Kunststoff, Elastomer, Gummi, Silikon, ein Luftpolster, Gelpolster, Flüssigkeitspolster oder schlauchartiges Material. Das weichere Material dient beispielsweise zur Erhöhung des Tragekomforts, kann auch zur Abdichtung der Kappe zur Haut (Dichtelement) eingesetzt werden. Das weichere Material, insbesondere Gummi und Silikon, kann als Haftelement oder rutschhemmendes Element auf der Haut dienen.

Die Dicke der Wandung der Kappe hängt vom Anwendungsfall und dem gewählten Material ab. Dabei spielt die gewünschte Festigkeit eine Rolle. Bei Verwendung von Kunststoff (z.B. Polyethylen oder Polypropylen) liegt die Wanddicke der Kappe im Allgemeinen im Bereich von 0,1 bis 3mm, vorzugsweise 0,1 bis 1,5 mm, insbesondere 0,1 bis 1 mm. Vorteilhaft ist eine Wandung mit Bereichen unterschiedlicher Dicke. Beispielsweise kann die Kappe in Bereichen eine stärkere, starke oder verstärkte Wandung aufweisen, die der Kappe größere Formstabilität verleiht oder sogar einen starren oder festen Bereich darstellt, und in einem oder mehreren Bereichen oder Zonen eine dünnere oder dünne Wandung, die flexibel, verformbar oder dehnbar ist. Flexible Zonen der Kappe werden beispielsweise durch falten-, balg- oder fugenartige Bereiche geschaffen. Flexible Zonen oder ringartige Bereiche werden vorteilhaft im Bereich oder nahe des Randes oder der Grundfläche der Kappe angeordnet.

Die Größe der Kappe ist in der Regel abhängig von der abzudeckenden Fläche der Wunde. Die Grundfläche der Kappe ist im Allgemeinen mindestens so groß wie die abzudeckende Wundfläche. Die Größe der Kappe kann ferner von den anatomischen Gegebenheiten und der Art der Wunde oder betroffenen Hautareals abhängen. Die Höhe der Kappe kann variiert werden und liegt beispielsweise im Bereich von 1 mm bis 10 cm, vorzugsweise im Bereich 3 mm bis 6 cm, insbesondere 1 bis 5 cm. Bei vielen Anwendungen ist ein großes Volumen der Kappe günstig.

Die Kappe ist je nach Anwendung ohne Öffnung oder mit einer oder mehreren Öffnungen in der Wandung versehen. Die Öffnungen können gleichartig oder unterschiedlich in Form und Größe sein. Öffnungen sind z.B. Rundöffnungen oder Schlitze. Vorteilhaft sind Öffnungen für die Lüftung (Belüftungs- und Entlüftungsöffnungen). Sie sorgen für eine gute Belüftung und Dampfdurchlässigkeit und ein günstiges Klima im Innenraum der Kappe. Alternativ ist der Einsatz von luftdurchlässigem Material (z.B. Gewebe, Gitter, Geflecht, offenporiges Material) oder von Kunststoff- oder Drahtkörben als Kappe vorteilhaft. Es können auch eine oder mehrere Öffnungen für andere Zwecke, beispielsweise zur Medikament-Applikation, vorgesehen werden. Die Öffnungen, Schlitze oder Aussparungen in der Wandung der Kappe weisen jeweils eine offene Fläche auf, die weniger als 50 Prozent, vorzugsweise weniger als 30 Prozent, besonders bevorzugt weniger als 10 Prozent und insbesondere weniger als 5 Prozent der Grundfläche der Kappe beträgt.

Eine für eine Medikament-Applikation vorgesehene Kappe verfügt in der Regel über eine oder mehrere Öffnungen oder Kanäle für die Zuführung eines Medikamentes zur Wunde. Vorteilhaft werden gasförmige Medikamente, Aerosole oder Sprays eingesetzt.

Die Kappe kann funktionalisiert werden. Beispielsweise können an der Innenseite der Kappe eine oder mehrere Einlagen (z.B. Kissen) für die Aufnahme von Feuchtigkeit, Blut oder Wundsekret angebracht werden. Vorteilhaft sind beispielsweise Einlagen mit einem so genannten Superabsorber. Einlagen können auch als Wirkstoffspender dienen.

Vorteilhaft ist die Kappe als Einwegartikel ausgebildet. Als Material zur Herstellung der Einweg-Kappe wird beispielsweise Pappe oder Kunststoff, insbesondere ein thermoplastischer Kunststoff wie Polyethylen, Polypropylen, Polyester (z.B. PET) oder Polystyrol verwendet.

Besonders vorteilhaft sind austauschbare oder auswechselbare Kappen. Ausgetauscht oder ausgewechselt wird in der Regel die ganze Kappe. So können Kappen verschiedenster Gestalt, Größe, Funktionalität, Stabilität, Materialbeschaffenheit oder sonstiger Eigenschaften je nach Anwendungsfall oder Situation eingesetzt und insbesondere beliebig mit einem Träger kombiniert werden. Beispielsweise können speziell für den Transport oder ähnliche Situationen vorgesehene Kappen, die z.B. eine höhere Festigkeit und Formstabilität aufweisen, gegen eine Kappe mit weicherer Beschaffenheit und höherem Tragekomfort ausgetauscht werden. Weiterhin ist der Austausch bei Verschmutzung der Kappe sehr vorteilhaft. Verschmutzte Kappen können ohne weiteres gegen neue Kappen ausgewechselt werden. Auswechselbare Kappen sind vorteilhaft mit Klettband, Klettbandabschnitten oder einem lösbaren Klebematerial (z.B. doppelseitiges Klebeband) als Befestigungsmittel ausgestattet.

Der Träger dient als Halter, zur Befestigung, Fixierung oder Positionierung der Kappe. Der Träger ist beispielsweise aus einem flexiblen Material wie Textil, Kunststoff (z.B. Elastomer), Kunstofffolie, Papier oder papierartigem Material. Der Träger ist in der Regel ein nichtadhäsiver (das heisst ohne Klebemittelschicht zur Befestigung am Körper), flächiger Träger. Ein flächiger Träger ist ein flächiges Gebilde, insbesondere ein am Körper tragbares Teil (tragbarer Träger). Nichtadhäsive, flächige Träger sind beispielsweise Kleidungsstücke, Bekleidungsteile, Unterbekleidungsteile, unmodifizierte (normale) oder modifizierte Unterhosen oder unmodifizierte oder modifizierte Windeln, Strumpfhosen, Kompressionsstrumpfhosen, Strümpfe oder Handschuhe. Die Befestigung des Trägers am Körper erfolgt im Wesentlichen ohne ein Klebemittel oder ohne eine Klebeschicht. Der Träger ist an der Fläche, die in der Anwendung an der Haut angelegt wird, also zur Körperseite, ohne Klebe- oder Adhäsivschicht. Der Träger wird also im Wesentlichen mit Hilfe mechanischer Kräfte am Körper befestigt und ist kein Pflastermaterial.

Bei einer Befestigung der Kappe an einem Träger wird z.B. der gesamte Rand, ein die Grundfläche der Kappe umlaufender Rand oder Randbereich, gegebenenfalls mit kleinen oder grösseren Unterbrechungen, oder eine oder mehrere Randflächen der Kappe mit dem Träger verbunden. Eine punktuelle Befestigung eines Trägers am Rand der Kappe mit insgesamt einem oder zwei Befestigungspunkten am Kappenrand ist in der Regel ungeeignet, da eine ausreichende Fixierung der Position der Kappe nicht gewährleistet und ein Wundschutz unzureichend ist.

Das Befestigungsmittel dient in der Regel zur Befestigung der Kappe auf einem Träger. Das Befestigungsmittel kann je nach Anwendungsfall lösbar oder unlösbar sein. Das Befestigungsmittel dient z.B. einer mechanischen, physikalisch oder chemisch haftenden Verbindung oder Befestigung. Das Befestigungsmittel ist beispielsweise ein selbst haftendes (z.B. Klettband) oder selbst klebendes Material und kann am Rand, an der Innenfläche oder Aussenfläche der Kappe angebracht oder integriert sein. Besonders vorteilhaft ist der Träger oder Teile des Trägers aus einem Material, auf dem Klettband oder klettbandartiges Material direkt haften. Auch kann eine Haftung durch van-der-Waals-Kräfte erfolgen. Solche Befestigungsmittel umfassen biomimetrische Materialien, die die Hafteigenschaften von Gecko-Füssen nachahmen.

Die Kappe kann mit der Innenseite mit dem Träger über ein oder mehrere Befestigungsmittel verbunden sein. Beispielsweise sind Befestigungsmittel auf der Innenfläche der Kappe angeordnet, z.B. Klettbandpunkte oder Klebe-Pads.

Die Kappe wird vorzugsweise mit Hilfe des Trägers mittelbar oder unmittelbar am Körper über der Wunde positioniert und in der gewünschten Position gehalten oder fixiert. Der Träger kann im Bereich der Unterseite der Kappe mit der Kappe in Verbindung stehen. Dies setzt eine Befestigung der Kappe an dem Träger nicht voraus. Eine permanente oder lösbare Befestigung der Kappe am Träger wird bevorzugt. Eine Befestigung, die einen Austausch oder die Abnahme der Kappe erlaubt, wird besonders bevorzugt. Vorteilhaft ist die Kappe als ein von dem Träger abnehmbares oder leicht entfernbares und wieder anbringbares Teil ausgebildet. Ein reversibles Entfernen und Anbringen der Kappe an dem Träger wird durch ein oder mehrere geeignete Befestigungsmittel erreicht. Ein bevorzugtes Befestigungsmittel ist Klettband oder Klettbandmaterial, das wie bei einem Klettverschluss zur lösbaren Befestigung der Kappe am Träger eingesetzt wird. Bei dieser Befestigung werden in der Regel Kappe und Träger mit komplementären Klettband-Elementen ausgestattet. Vorteilhaft wird ein Träger eingesetzt, dessen Material so beschaffen ist, dass an dessen Oberfläche Klettband haftet. Bei einem solchen Träger sind zusätzliche Klettband-Elemente nicht erforderlich. Die Kappe wird in der Regel direkt, also ohne Zwischenstück, am Träger befestigt.

Eine leichte Abnehmbarkeit und Wiederanbringbarkeit der Kappe ist vorteilhaft für eine Inspektion der Wunde und eine Wundbehandlung bei einem Träger mit einer Öffnung oder Aussparung im Bereich der Wunde.

Vorteilhaft ist eine Austauschbarkeit der Kappe in vielerlei Hinsicht. Neben einer einfachen Austauschbarkeit einer verschmutzten Kappe durch eine gereinigte, gewaschene oder neue Kappe - besonders praktisch sind hier als Einwegartikel ausgeführte Kappen - ist der Austausch durch Kappen mit unterschiedlichen Eigenschaften, z.B. unterschiedlicher Festigkeit oder Gasdurchlässigkeit, sehr nützlich, wobei ein Wechsel des Trägers nicht erforderlich ist. Dadurch kann die Wundabdeckung leicht sich ändernden Situationen angepasst werden.

Die Wundabdeckung gemäß der Erfindung gestattet die Heilung einer Wunde ohne Materialkontakte (kontaktfreie Wundabdeckung). Es kommt nicht zu einem Anhaften oder Verkleben der Wunde mit dem Verband und einem Aufreißen der Wunde bei der Entfernung des Verbandmaterials. Beispielsweise kommt es nicht mehr zu einem Aufreißen der Wunde des frisch operierten Penis. Ferner bietet die Wundabdeckung Schutz vor einem accidentellen Zurückdrücken von Hoden in die Leiste bei operativ aus der Leiste in das Skrotum (Hodensäckchen) verlagerten Hoden. Weiters werden durch die Wundabdeckung Umwelteinflüsse abgeschirmt. Der Heilungsprozess wird somit begünstigt. Andererseits vermeidet die Wundabdeckung eine Verschmutzung der Kleidung durch Blut oder Wundabsonderungen. Die Kappe dient also insbesondere der komplikationsmindernden offenen Wundbehandlung durch die kontaktfreie Abdeckung einer Wunde und verhindert die externe, direkte Keimkont amination durch die Vermeidung der Berührung der Wunde mit der Kleidung, einem Verband oder einem anderen Material.

Vorteilhaft werden durch die Kappe mechanische postoperative Probleme durch Druck- oder Scherkrafteinwirkungen von außen verhindert. Durch eine formstabile Kappe, insbesondere durch eine feste Kappe (z.B. einer für den Transport eines Patienten angepassten Kappe), wird die Wunde vor Druck- und mechanischer Krafteinwirkung geschützt, wie sie von einem angelegten Sicherheitsgurt ausgehen.

Bevorzugte Wundabdeckung ist eine Penis-Wundabdeckung, insbesondere für Kinder oder Säuglinge. Die Penis-Wundabdeckung ist insbesondere vorteilhaft für Säuglinge und Kinder, die nach Operationen am Penis wie z.B. Circumcision, Vorhaut erhaltender Präputialplastik bei Vorhautverengungen (Phimose) sowie weiteren Operationen, auf Grund ihres gesteigerten Bewegungsdranges und fehlender Einsicht für die erforderliche Schonung sowohl einen mechanischen Schutz der Operationswunde für 2-3 Wochen gegen unkontrollierte Bewegungen sowie gegen Keimkontamination durch Verunreinigung-, Stuhl- und Urinkontakt benötigen.

Die Penis-Wundabdeckung umfasst einen am Unterleib tragbaren Träger wie eine Textilunterhose oder Windel, der vorteilhaft im Genitalbereich weit ausgeschnitten ist. Der Ausschnitt wird vorzugsweise von einem eingenähten schmalen Klettband gesäumt. Als Kappe wird beispielsweise ein Kunststoffkörbchen mit einem flachen Rand verwendet, der mit einem Klettband als komplementärem Gegenstück zum Klettband am Träger ausgestattet ist. Der durch die Klettbänder gebildete Klettverschluss erlaubt eine einfache Befestigung der Kappe unter Vermeidung der Berührung der Wunde. Durch einfaches Anlegen der Kappe wird der Ausschnitt am Träger wie Unterhose oder Windel geschlossen. Die Kappe trägt vorteilhaft zahlreiche Öffnungen zur freien Luftzirkulation.

Träger (z.B. am Körper tragbares Teil, Textilteil) und Kappe (z.B. Kunststoffteil, Kunststoffschale) der Penis-Wundabdeckung werden vorteilhaft in unterschiedlichen Größen kindadaptiert hergestellt und sind untereinander den anatomischen Verhältnissen angepasst kombinierbar. Die anatomiegerechte Kombination unterschiedlicher Trägergrößen (z.B. Textilgrößen) mit unterschiedlichen Größen der Kappe (Kunststoffkörbchengrößen) ist möglich und angezeigt. Ein Verbandwechsel und lokale Penisbehandlung sind durch einfache Abnahme der Kappe möglich. Eine abnehmbare Kappe bietet den weiteren Vorteil, dass Kappe (z.B. Kunststoffteil) und Träger (z.B. Textilteil) getrennt gewaschen werden können.

Das früher (ohne die Penis-Wundabdeckung) häufig beobachtete und zu erneuten, wiederholten Arztbesuchen führende accidentelle und äusserst schmerzhafte Anstossen des frisch operierten Penis bei den normalen Bewegungen des täglichen Lebens sowie beim Spiel oder Sport mit sequenziellen Hämatombildungen (Bildung von Blutergüssen), Nachblutungen, mechanischen Reizungen und Infektionen stellt für Kinder mit ihrem gesteigerten Bewegungsdrang ein wesentliches Problem nach der Operation dar. Diese Problematik wird durch die Penis-Wundabdeckung gänzlich beseitigt.

Die Kappe verhindert zuverlässig die schmerzhafte Verklebung der frischen Penis-/Glanswunde mit der Unterhose oder dem Verband sowie komplizierende Nachblutungen im Zusammenhang mit der erforderlichen Ablösung der Wundadhärenz.

Darüber hinaus wird eine frühere, schmerzfreie und Komplikationen verhindernde Mobilisierung der Kinder sowie eine frühere Kindergarten-, Schul-, und Sportfähigkeit erreicht.

Eine weitere Variante ist ein windelartiger Wundverband mit Kappe. Die Kappe ist z.B. im Schrittbereich eines windelartigen Trägers angeordnet. Der Träger ist, z.B. eine gewöhnliche Windel, insbesondere eine Wegwerf-Windel oder Einmal-Windel. Die Kappe wird beispielsweise im Aussenbereich (über der Windel, an der Aussenhülle der Windel) positioniert. Der Träger (die Windel) kann im Bereich der Kappe eine vollständige oder teilweise Materialaussparung aufweisen. Die Kappe kann mit einem Flüssigkeit aufnehmendem Material ausgestattet werden. Die Kappe des windelartigen Wundverbandes dient vorzugsweise zur Abdeckung von Wunden im Genitalbereich, z.B. bei Penisbeschneidungen, insbesondere bei Säuglingen. Der windelartige Wundverband mit Kappe wird nicht nur bei Säuglingen, sondern auch bei älteren Windelträgem und Inkontinenz verwendet. Der windelartige Wundverband mit Kappe im Genitalbereich ist eine Variante der Penis-Wundabdeckung.

In einer besonderen Gestaltungsform ist eine Kappe, die auf der Innenseite mit einem oder mehreren Befestigungselementen oder Befestigungsmitteln versehen ist, mit der Aussenseite einer Windel (z.B. übliche Einmal-Windel) verbunden. Die Windel wird durch das oder die Befestigungsmittel an der Kappeninnenseite gehalten und behält so in diesem Bereich eine Wölbung, die einen Materialkontakt mit der Wunde verhindert. Besonders vorteilhaft ist die Verwendung von Klettband-material als Befestigungsmittel, das in der Regel an dem Windelmaterial von Einmal-Windeln direkt haftet. Beispielsweise wird eine Windel an eine mit Klettband oder Klettbandmaterial (z.B. Befestigungselemente, Pads) versehene Kappe angedrückt, wodurch die Kappe befestigt wird. Die Kappe, insbesondere Aussenkappe, hält die Windel in der gewölbten Form und ist leicht abnehmbar.

Vorteilhaft ist die Verwendung einer Kappe auf einem tragbaren Träger zur Wundabdeckung, zur Abdeckung von Operationswunden, als medizinische Abdeckung, als Hilfsmittel zur Behandlung der Haut oder zur Medikament-Applikation, Wirkstoff-Applikation oder Kosmetikmittel-Applikation am Körper, als Wundschutz, als mechanischer Wundschutz, als Schutzeinrichtung, zur Wundprophylaxe oder als Kammer zur lokalen Behandlung der Haut mit einem Gas oder Gasgemisch bei Normaldruck oder Überdruck. Besonders vorteilhaft ist die Verwendung einer Kappe mit tragbarem Träger, zur Wundabdeckung und/oder zu einem mechanischen Wundschutz bei Kindern und Säuglingen.

Besonders vorteilhaft ist die Verwendung einer Wundabdeckung mit Kappe auf tragbarem Träger zur skrotalen Positionssicherung der Hoden im Hodensäckchen nach Hodenoperationen, insbesondere nach Hodenverlagerungen wegen eines angeborenen Hodenhochstands. Zu den postoperativen Problemen und bekannten Komplikationen gehört das Frührezidiv (Wiederzurückgleiten) der frisch in das Hodensäckchen operativ verlagerten Hoden durch mechanische Einflüsse verursacht durch Dritte (z.B. zu eng angelegte Windeln, eng ansitzender Body-Anzug, falsches Tragen des Kindes) oder verursacht durch das Kind selbst, beispielsweise beim Klettern oder Rutschen oder durch Sturz. Die Kappe wird vorteilhaft mit einer Einbuchtung für den Penis in der Form modifiziert. Die Form wird beispielsweise so gestaltet, dass die Urinableitung über eine Ausbuchtung am mittleren Rand erfolgt und die Wunde ohne Urinkontakt trocken bleibt.

Vorteilhaft ist die Verwendung einer Kappe auf einem tragbaren Träger zur Medikamenten-Applikation, Wirkstoff-Applikation oder Kosmetikmittel-Applikation am Körper, vorzugsweise zur Behandlung von Hautarealen oder äusseren Körperstellen mit einem Gas, Gasgemisch, Aerosol oder Spray, insbesondere zur Behandlung mit einem als Gas, Gasgemisch oder Aerosol verabreichten Medikament bei Normaldruck oder Überdruck. Dabei bildet die Kappe eine Kammer für das Medikament über dem Hautareal. Vorteilhaft ist die Kappe so beschaffen, dass mit dem Gas, Gasgemisch oder Aerosol ein Überdruck, vorzugsweise ein leichter Überdruck, in der Kappe erzeugt werden kann. Die Kappe wird bei dieser Verwendung insbesondere als eine Art Druckkammer eingesetzt. Vorteilhaft werden als gasförmiges Medikament reiner Sauerstoff oder Gasgemische mit einem Sauerstoffgehalt von über 30 Volumenprozent, vorzugsweise von über 50 Volumenprozent, insbesondere von über 80 Volumenprozent, oder ein Stickstoffmonoxid (NO) enthaltendes Gasgemisch eingesetzt. Vorteilhaft ist somit die Verwendung eines gasförmigen oder aerosolartigen Medikamentes in einer Kappe als Behandlungskammer auf einem Hautareal, insbesondere zur Behandlung von Wunden oder eines von einer Krankheit, einem Erreger oder Pilz befallenen Hautareals. Vorteilhaft wird ein gasförmiges oder aerosolartiges Medikament, insbesondere Sauerstoff, ein sauerstoffangereichertes Gasgemisch oder ein NO-haltiges Gasgemisch, zur lokal begrenzten, äusserlichen Behandlung von Wunden, offenen Beinen, Mykosen, Dermatitis oder Schuppenflechte oder präventiv zum Schutz der Haut eingesetzt.

Vorteilhaft wird die Kappe insbesondere zur Applikation von Medikamenten oder Medikationen zur Gefässerweiterung, z.B. ein NO-haltiges Gasgemisch, verwendet, insbesondere bei Durchblutungsstörungen, chronische Hautulcera, Transplantationen oder oberflächlichen Gefässanastomosen.

Die Wundabdeckung wird vorteilhaft als Wundschutz, Hautschutz oder Körperschutz verwendet, insbesondere als Schutz vor mechanischen Einwirkungen von Wunden, Haut-oder Körperarealen. Ferner dient die Wundabdeckung als Schutzeinrichtung zum Schutz vor physikalischen oder chemischen Einflüssen, insbesondere vor Exkrementabgängen, an den mit der Kappe abgedeckten Körperstellen.

Die Erfindung wird anhand von Beispielen in der Zeichnung erläutert.

Es zeigen:
Fig.1: Schema einer Wundabdeckung im Querschnitt;
Fig. 2: Schema einer Wundabdeckung in der Draufsicht;
Fig. 3: Schema einer Wundabdeckung mit austauschbarer Kappe im Querschnitt;
Fig. 4: Schema einer Wundabdeckung mit aufklappbarer Kappe im Querschnitt;
Fig.5: Schema einer Wundabdeckung mit einer Kappe für die Medikamentenapplikation im Querschnitt;
Fig. 6: Schema einer Wundabdeckung mit aussen an einem Träger angeordneter Kappe im Querschnitt (Detailansicht im Bereich der Kappe);
Fig. 7: Schema einer Kappe mit Verstärkung in der Draufsicht;
Fig. 8: Schema einer Wundabdeckung für den Genitalbereich (Vorderansicht);
Fig. 9: Schema eines Trägers einer Wundabdeckung für den Genitalbereich ohne Kappe (Vorderansicht);
Fig. 10: Schema einer Windel mit Kappe (Draufsicht, Aussenseite).

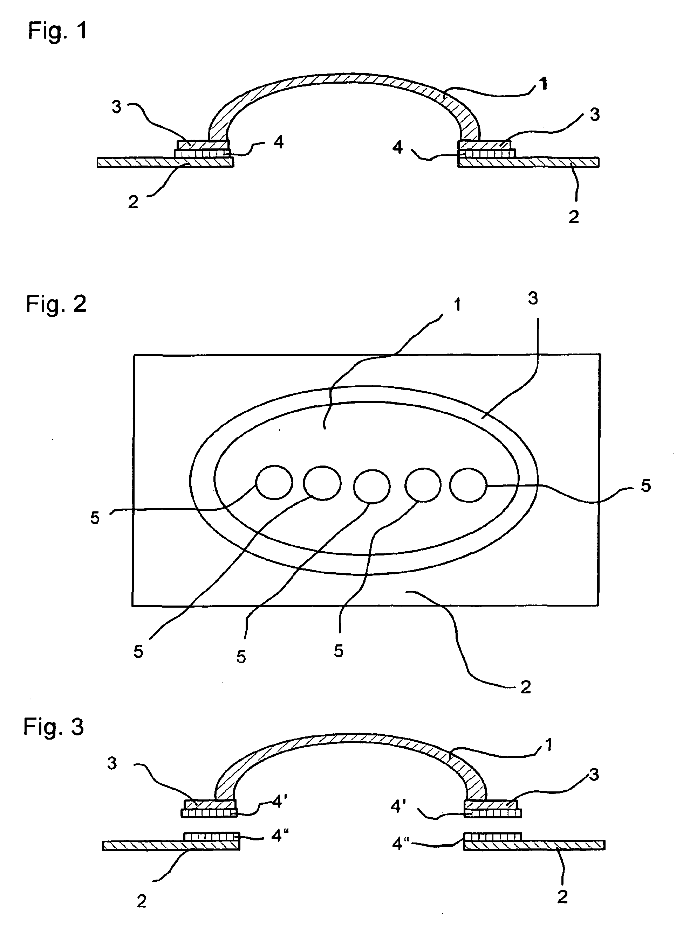

Das allgemeine Schema in Fig. 1 zeigt ein Beispiel einer Wundabdeckung mit aufgesetzter Kappe 1 im Querschnitt. Die Wundabdeckung umfasst eine Kappe 1 (Wundabdeckung) mit einem optionalen Rand 3 (Bestandteil der Kappe), einen Träger 2 und ein Befestigungsmittel 4. Die Kappe 1 ist formstabil oder weitgehend formstabil, was durch geeignete Wahl des Materials oder durch eine Verstärkung oder Verstrebung des Materials erreicht wird. Das Material ist z.B. Kunststoff. Die Kappe ist schalenförmig oder korbartig aufgebaut und hat z.B. eine ovale Grundfläche. Die Kappe 1 kann eine oder mehrere seitliche Ausbuchtungen oder Einbuchtungen haben. Der Träger 2 dient als Halter, zur Befestigung, Fixierung oder Positionierung der Kappe über der Wunde. Der Bereich der Wunde bleibt frei, das heißt die Wunde hat keinen direkten Kontakt zu Träger 2 oder Kappe 1 (kontaktfreie Wundabdeckung). Der Träger ist beispielsweise aus einem flexiblen Material wie Textil, Kunststoff (z.B. Elastomer), Kunstofffolie, Papier oder papierartigem Material. Der Träger 2 ist z.B. ein Kleidungsteil, vorzugsweise ein Unterbekleidungsstück, insbesondere eine Unterhose, ein Unterhemd, ein Mieder, ein Korsett oder eine Windel, die im Bereich der Kappenanordnung modifiziert sein können. Das Befestigungsmittel 4 kann beispielsweise eine Verklebung, ein Klebefilm, ein oder mehrere mechanische Befestigungen (z.B. Knopf, Clip, Steckverbindung, Rastverbindung, Klettverschluss, Schnappverschluss), eine magnetische Befestigung, eine Verschweißung oder Schweißpunkte sein. Das Befestigungsmittel 4 kann z.B. am Rand 3 angeordnet und durchlaufend sein oder auch durch zwei oder mehrere an oder auf dem Rand 3 verteilte Mittel gebildet werden. Das Befestigungsmittel 4 kann zur permanenten oder zur lösbaren Befestigung ausgelegt sein. Ein lösbares oder leicht lösbares Befestigungsmittel 4 wird insbesondere bei austauschbaren, abnehmbaren, klappbaren oder zur Grundfläche aufklappbaren Kappen 1 eingesetzt.

Die Kappe ist durchsichtig oder undurchsichtig. Eine transparente oder teilweise transparente Kappe kann vorteilhaft sein, z.B. für eine einfache visuelle Kontrolle der Heilung der Wunde.

Die Wundabdeckung von Fig. 1 wird als vereinfachtes Schema in der Draufsicht in Fig. 2 gezeigt. Im gezeigten Beispiel sind mehrere Öffnungen 5 an der Kappe 1 angeordnet. Die Öffnungen in der Wandung der Kappe 1 können unterschiedliche Formen haben, beispielsweise rund, oval, eckig, schlitzförmig. Die Öffnungen 5 werden z.B. in einer, zwei oder mehreren Reihen in Quer- oder Längsrichtung oder in beiden Richtungen an der Kappe 1 angeordnet. Die Öffnungen 5 dienen z.B. einer Belüftung. Es können Öffnungen 5 verschiedener Grösse und Form kombiniert werden, wobei die Öffnungen verschiedene Funktionen haben können, wie Belüftung, Sichtfenster, Zufuhr oder Entnahme. Es kann vorteilhaft sein, Öffnungen nur in bestimmten Kappenbereichen anzuordnen. Beispielsweise bei Wundabdeckungen in Penis-Nähe bei Kleinkindern (z.B. Abdeckung des Skrotum oder des Skrotum und einer Leiste; dabei ist der Penis ausserhalb der Kappe 1) vermeidet eine geschlossene Wandung auf der Penis zugewandten Seite der Kappe ein Eindringen von Urin in die Kappe, der sonst zur Wunde gelangen würde.

Der optionale Rand 3 der Kappe 1 ist z. B. abgeflacht. Der Rand 3 kann wie hier gezeigt nach aussen oder nach innen gerichtet sein, je nach den Erfordernissen. Der Rand 3 kann durchgehend oder unterbrochen sein. Der Rand 3 ist z.B. auf dem Träger 2 direkt (z.B. geklebt oder genäht) oder über ein oder mehrere Befestigungsmittel 4 (in Fig. 2 nicht gezeigt) befestigt, wobei die Befestigung permanent oder lösbar sein kann. Eine lösbare Befestigung, die einen leichten Austausch der Kappe 1 erlaubt, wird beispielsweise durch Klettband als Befestigungsmittel 4 realisiert, das jeweils an Rand 3 und dem Träger 2 an entsprechender Stelle an den sich gegenüberliegenden Seiten befestigt (z. B. verklebt oder vernäht) ist. Diese Klettverschluss-artige Befestigung ist sehr vorteilhaft für eine vorübergehende Entfernung der Kappe 1 vom Träger für eine Versorgung oder Inspektion der Wunde. Der gezeigte Träger 2 ist als als Ausschnitt eines grösseren Teiles (z.B. als Teil eines Kleidungsstückes) zu betrachten.

In Fig. 3 wird eine besondere Ausführungsform des Befestigungsmittels 4 von Fig. 1 veranschaulicht. Das Befestigungsmittel 4 umfasst hier die Befestigungsteile 4' und 4". In dem gezeigten Beispiel wirken die Befestigungsteile 4' und 4" zusammen oder komplementär. Diese Art der Befestigung liegt beispielsweise bei einem Klettverschluss vor, wo übereinander liegende komplementäre Klettbänder ineinander greifen. Die Befestigungsteile 4" können entfallen, wenn der Träger 2 eine Oberflächenbeschaffenheit aufweist, auf der die Befestigungsteile 4' haften. Dies ist z.B. der Fall bei Klettbandmaterial als Material für die Befestigungsteile 4' und einem Träger 2 mit einer rauen Vlies- oder Textil-Oberfläche.

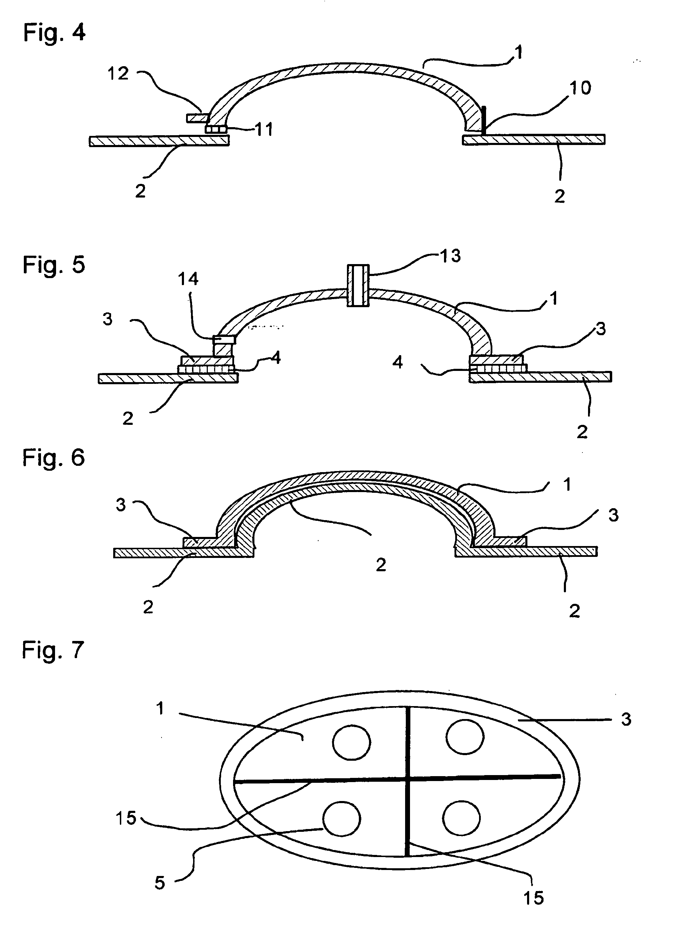

Im Beispiel von Fig. 4 ist die Kappe 1 als aufklappbarer Deckel ausgeführt. Die Kappe 1 ist über eine Art Scharnier 10, z.B. eine Folie oder Trägermaterial, klappbar am Träger 2 angeordnet. Als Verschluss dient z.B. ein punktueller Klettbandverschluss 11 (vereinfacht dargestellt). Vorteilhaft ist ein Griffteil 12 an der Kappe 1. Hier gibt es viele Ausführungs--möglichkeiten.

Fig. 5 zeigt eine weitere Variante der Wundabdeckung von Fig. 1. Die Kappe 1 ist hier als Volumenaustauschkammer ausgebildet. Durch eine oder mehrere Zuleitungen 13 kann ein therapeutisch wirksames Gas, Gasgemisch (medizinisches Gas), Dampf oder Aerosol dem Innenraum unter der Kappe 1 zugeführt werden. Durch eine oder mehrere Öffnungen oder Kanäle 14 kann das eingebrachte Volumen entweichen (Spüleffekt) oder durch Verschluss der Öffnungen in der Konzentration gehalten werden. Beispielsweise kann reiner Sauerstoff oder ein sauerstoffreiches Gasgemisch als medizinisches Gas eingesetzt werden, die Feuchtigkeit der Wundumgebung vermehrt werden, die Wunde getrocknet oder Medikationen zur Gefässerweiterung appliziert werden (Anwendung bei Durchblutungsstörungen, chronische Hautulcera, Transplantationen, oberflächliche Gefässanastomosen). Die Öffnungen 13 und 14 können mit Ventilen oder steuerbaren Ventilen ausgestattet werden.

Es können mehrere Kappen 1 an einem Träger 2 angeordnet werden. Eine Anordnung von mehreren Kappen 1 wird z.B. bei multiplen kleinflächig betroffenen Hautarealen eingesetzt. Vorteilhaft sind örtlich frei platzierbare Kappen auf einem Träger, der an den Wunden offen ist und die Öffnungen durch Befestigung der Kappen abgedeckt werden. Sehr vorteilhaft ist, wenn die Kappen mit einem Klettband oder entsprechend haftendem Material als Befestigungsmittel ausgestattet sind, so dass die Kappen auf dem Träger haften, z.B. ein für eine Kletthaftung geeignetes Vlies. Beispielsweise sind Kappen mit Befestigungsstellen (Klettbandabschnitte oder-punkte) auf der Innenseite der Kappe beliebig auf einem solchen Träger platzierbar.

In Fig. 6 ist eine aussen an einem Träger 2 befestigte Kappe 1 (z.B. windelartiger Wundverband mit Aussenkappe) im Querschnitt und stark vereinfacht gezeigt. Die Kappe 1 (z.B. eine Kunststoff-Schale) mit optionalen Rand 3 ist auf dem als Träger 2 (z.B. Windelmaterial oder Unterhose) angeordnet, gewöhnlich im Schrittbereich oder Genitalbereich. Die Kappe kann aber auch an anderen Stellen der Windel zur Wundabdeckung angeordnet werden. Die Kappe 1 ist z.B. unterhalb der Aussenhülle (nicht gezeigt) der Windel angebracht. Beispielsweise ist die Kappe 1 ganzflächig, auf Teilflächen oder punktuell auf den Träger 2 (z.B. Windelmaterial oder Unterhose) aufgeklebt (z.B. mit doppelseitigem Klebeband, Klebebandabschnitten oder Klebepunkten) oder mechanisch befestigt (z.B. genäht, geclipst oder mittels Klettbandmaterial). Besonders vorteilhaft ist die Anordnung von Befestigungsstellen, Befestigungselementen oder Befestigungspunkten (z.B. Klebepunkte oder Klettbandpunkte) auf der Innenseite von Kappe 1. Klettbandmaterial haftet in der Regel auf der rauen Aussenseite von gewöhnlichen Einmal-Windeln oder Wegwerfwindeln. Daher lässt sich eine solche Windel sehr leicht für einen Wundschutz anpassen, indem der betreffende Windelbereich (Träger 2) in die Kappe 2, die mit Befestigungsmittel oder Befestigungselementen auf der Innenseite ausgestattet ist, gedrückt wird. In diesem Beispiel wird das Windelmaterial bzw. die Windel (Träger 2) so von der Kappe 1 im Bereich der Wunde auf Abstand zur Haut gehalten. Die Kappe 1 kann auch ein Bügel sein.

Eine mit Streben 15 verstärkte Kappe 1 der Wundabdeckung wird in Fig. 7 gezeigt (Draufsicht). Eine oder mehrere Streben 15 verstärken die Kappe 1, die beispielsweise aus Textil, etwa ein Gewebe oder Vlies, oder aus einem anderen weniger oder gar nicht formstabilen Material besteht. Die Streben 15 bilden z.B. ein Gitter. In der Kappe 1 sind je nach Anwendungsfall eine oder mehrere Öffnungen 5 angeordnet. Die Kappe 1 wird über den Rand 3 mit dem Träger 2 (hier nicht gezeigt) verbunden.

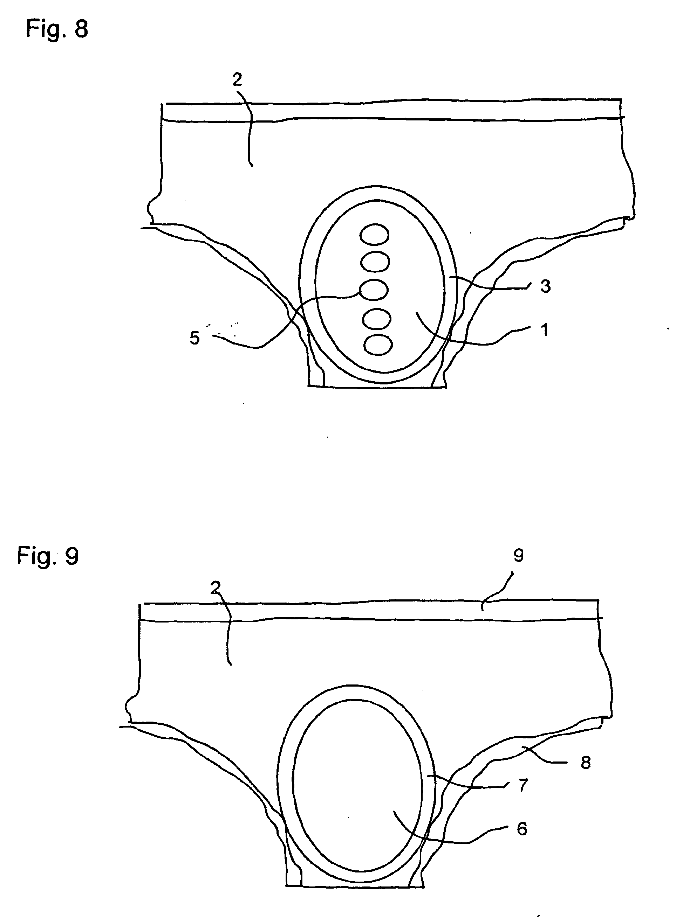

Fig. 8 zeigt die Vorderansicht eines Kleidungsstückes, eines Unterbekleidungsstückes wie hier eine modifizierte Unterhose als Träger 2 für eine Kappe 1, die über den Rand 3 mit dem Träger 2 verbunden ist. Die Befestigung der Kappe 1 an dem Träger 2 erfolgt vorteilhaft in der in Fig. 3 dargestellten Weise mit Klettbändern (Klettverschluss). Die Kappe 1 weist vorteilhaft mehrere Belüftungsöffnungen 5 auf. Die Wundabdeckung wird besonders vorteilhaft zur Abdeckung des operierten Penis verwendet. Die Kappe 1 ist in der Grösse so bemessen, dass die Genitalien (Penis und Hoden) von dem Innenraum der Kappe aufgenommen werden können.

Der Träger 2 (Unterhose) von Fig. 8 ist in Fig. 9 ohne Kappe 1 dargestellt. Im Bereich des Penis bzw. der Genitalien ist die Öffnung 6 angeordnet. Die Öffnung 6 ist von einem Saum 7 umgeben, auf dem ein Klettband zur Befestigung der Kappe 1 aufgenäht ist. Entsprechend ist auf dem Rand 3 der Kappe 1 ein Klettband angeordnet, das z.B. angenäht ist. Als weitere Details der Unterhose (Träger 2) sind der Bund 9 und die Beinausschnitte 8 gezeigt.

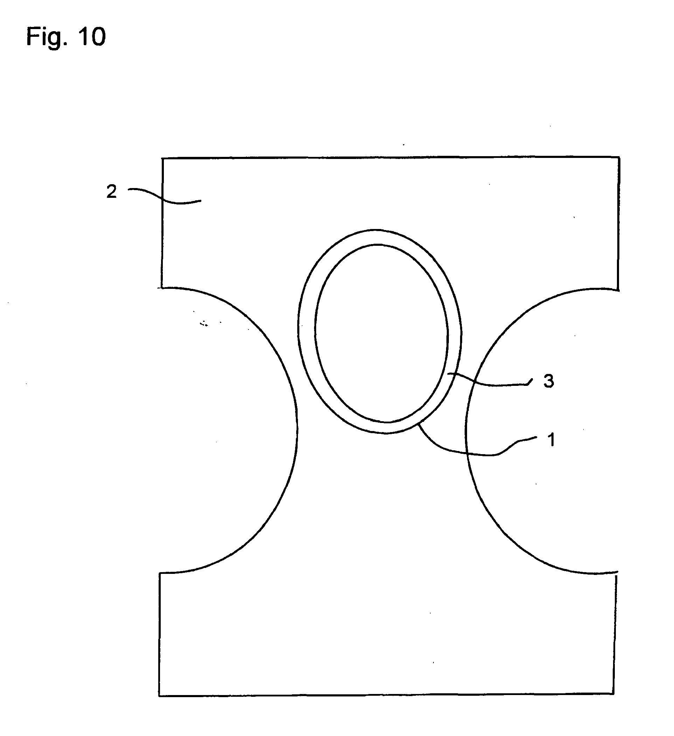

Eine weitere Ausführungsform des Wundverbandes ist in Fig. 10 dargestellt. Hier ist der Wundverband eine modifizierte Windel als Träger 2 mit einer Kappe 1, die optional einen flachen Rand 3 aufweist. Hier ist die Aussenseite der Windel in stark vereinfachter Darstellung zu sehen (Draufsicht). Der Träger 2 ist in der Regel wie eine gewöhnliche Windel für Säuglinge beschaffen. Gewöhnlich weist die Windel an der Innenseite ein feuchtigkeitsdurchlässiges Material in Kombination mit einem saugfähigen und Nässe bindendem Material (z.B. sog. Superabsorber) und ein Wasser undurchlässiges Material (z.B. Kunststofffolie) an der Aussenseite auf. Die Kappe 1 ist bei dem gezeigten Beispiel im Genitalbereich angeordnet. Die Kappe 1 wird z.B. im Aussenbereich der Windel (z.B. auf der Aussenseite der Windel) angeordnet. Die Windel kann im Bereich der Kappe eine Öffnung (Materialaussparung) oder eine partielle Materialaussparung (z.B. Fehlen einer Schicht wie Aussen- oder Innenmaterial) oder im Bereich der Kappe das gewöhnliche Material aufweisen. Es können eine oder mehrere Kappen 1, z. B. nebeneinander oder übereinander, an oder in der Windel, dem Träger 2, angeordnet werden. Die Kappe wird z.B. durch Kleben oder Nähen, mit Hilfe von Klebematerial wie Klebeband oder durch mechanische Mittel wie Clipse mit dem Träger 2 oder einem Teil des Trägers (z.B. eine Schicht oder Innen- oder Aussenhülle) verbunden. Die Kappe 1 hat vorzugsweise eine feuchtigkeits- und luftdurchlässige Struktur, was z.B. durch Öffnungen, ein netzartiger oder gitterartiger Aufbau erreicht wird. Im Innenraum der Kappe 1 kann zusätzlich ein saugfähiges oder feuchtigkeitsbindendes Material angeordnet werden, z.B. eine Schicht oder ein Kissen mit wasserabsorbierendem Material (z.B. so genannter Superabsorber).

Die Kappe 1 des windelartigen Wundverbandes ist in der Regel ein formstabiler, schalenartiger Hohlkörper. Sie dient dazu, einen Abstand von der Wunde (Penis) zur inneren Oberfläche der Windel zu halten. Vorteilhaft wird zugleich Urin in gewohnter Weise mit der Windel (Träger 2) aufgefangen.

Eine Windel mit Aussenkappe hat den Vorteil, dass keine harten (formstabilen) Strukturen Kontakt mit der weichen Babyhaut haben, andererseits besteht möglicherweise eine etwas grössere Gefahr des Verrutschens, da der Formträger (die Kappe 1) aussen sitzt.

Eine Windel mit Innenkappe (an der Innenseite einer Windel angebrachte Kappe) hat den Vorteil, dass der formstabile Anteil (die Kappe) den Abstand zum Penis sehr gut sichert. Möglicherweise aber die Urinableitung und Absorption im Vergleich zu einer gewöhnlichen Windel verschlechtert. Ausserdem könnte der Tragekomfort durch eine mit der weichen Babyhaut in Kontakt kommende Kappe beeinträchtigt werden.

Es können Aussen-, Zwischen- ( Kappe zwischen zwei Windellagen oder -schichten) und Innenkappe beliebig nebeneinander oder übereinander kombiniert werden. Beispielsweise kann eine weichere Innenkappe unter einer Aussenkappe angeordnet werden.

Die windelartigen Wundverbände sind ausser nach Beschneidungen bei zahlreichen Genitalerkrankungen mit Affektionen der Haut im Säuglingsalter wie z.B. der häufigen Windeldermatitis aber auch bei Mykosen wie z.B. der häufigen Candidamykose vorteilhaft einsetzbar.

Der windelartige Wundverband, also Windel als Träger 2 mit Kappe 1, wird besonders bevorzugt als Einmalartikel eingesetzt. Dabei werden beispielsweise gewöhnliche Windeln mit einer Kappe aus einer Kunststoffschale, insbesondere eine Kunststoffschale mit Öffnungen, oder einer Kappe aus einer netz- oder gitterartigen Struktur aus Kunststoff kombiniert.

## Patentansprüche

1. Wundabdeckung für eine kontaktfreie Abdeckung einer Wunde oder eines Hautareals mit mindestens einer formstabilen Kappe (1) und einem nichtadhäsiven, flächigen Träger (2), wobei der Träger (2) ein Kleidungsstück, ein Unterbekleidungsteil, eine Unterhose, ein Slip, eine Windel, eine Strumpfhose, eine Kompressionsstrumpfhose, ein Strumpf, ein strumpfartiges Teil, ein Handschuh oder eine tragbare Bandage ist und die Kappe (1) auf dem Träger (2) angeordnet oder befestigt ist.

2. Wundabdeckung nach Anspruch 1, **dadurch gekennzeichnet, dass** die Kappe (1) mit einem an der Grundfläche gelegenen Rand (3) auf dem Träger (2) angeordnet oder befestigt ist.

3. Wundabdeckung nach Anspruch 1 oder 2, **dadurch gekennzeichnet, dass** die Kappe (1) unlösbar, lösbar, entfernbar, abnehmbar oder austauschbar mit dem Träger (2) verbunden ist.

4. Wundabdeckung nach Anspruch 1 oder 2, **dadurch gekennzeichnet, dass** die Kappe (1) mit dem Träger (2) durch Vernähen, Verkleben, Verschweissen, Heften, Anheften, chemische oder physikalische Haftung, mechanische Wirkung, Klettband, eine Steckverbindung oder eine Einrastverbindung verbunden ist.

5. Wundabdeckung nach einem der Ansprüche 1 bis 4, **dadurch gekennzeichnet, dass** die Kappe (1) eine oder mehrere Öffnungen (5, 13, 14), eine oder mehrere verschließbare Öffnungen (5, 13, 14), eine oder mehrere Öffnungen (5, 13, 14) mit einem passiven oder aktiven Ventil, einen oder mehrere Schlitze oder ein oder mehrere Kanäle (13, 14) aufweist.

6. Wundabdeckung nach einem der Ansprüche 1 bis 4, **dadurch gekennzeichnet, dass** die Kappe (1) ein oder mehrere Einlagen, ein oder mehrere Auflagen, ein oder mehrere Dichtelemente im Randbereich (3), ein oder mehrere Haftelemente im Randbereich (3), ein oder mehrere Haftelemente oder Befestigungsmittel (4) auf der Innenfläche oder Außenfläche, ein oder mehrere rutschhemmende Elemente im Randbereich (3) oder ein oder mehrere Feuchtigkeits- oder Nässe-Absorber oder -binder aufweist.

7. Wundabdeckung nach einem der Ansprüche 1 bis 4, **dadurch gekennzeichnet, dass** die Kappe (1) ein oder mehrere Speicher- oder Abgabeeinrichtungen für feste, flüssige oder gasförmige Medikamente oder therapeutisch eingesetzte Stoffe oder Materialien, ein oder mehrere keimabtötende Stoffe oder eine Medikament-Zuführungseinrichtung aufweist.

8. Wundabdeckung nach einem der Ansprüche 1 bis 4, **dadurch gekennzeichnet, dass** die Kappe (1) ein oder mehrere Fenster oder strahlentransparente, strahlenfokusierende oder strahlenfilternde Bereiche, ein oder mehrere Funktionselemente, eine transparente, in Teilen oder teilweise transparente Wandung, eine undurchsichtige oder für Strahlen undurchlässige Wandung, eine Lüftungseinrichtung oder eine Gaszuführung aufweist.

9. Wundabdeckung nach einem der Ansprüche 1 bis 8, **dadurch gekennzeichnet, dass** der Träger (2) in dem Bereich, an dem die Kappe (1) angeordnet ist, eine Öffnung oder Aussparung (6) oder der Träger (2) ein oder mehrere rutschhemmende Elemente auf der dem Körper zugewandten Seite aufweist.

10. Verwendung mindestens einer formstabilen Kappe (1) mit einem oder mehreren Befestigungsmitteln (4) und einem nichtadhäsiven, flächigen Träger (2) als Mittel für eine kontaktfreie Abdeckung eines Hautareals oder eine kontaktfreie Wundabdeckung, wobei die Kappe (1) auf dem Träger (2) angeordnet oder befestigt ist und der Träger (2) ein Kleidungsstück, ein Unterbekleidungsteil, eine Unterhose, ein Slip, eine Windel, eine Strumpfhose, eine Kompressionsstrumpfhose, ein Strumpf, ein strumpfartiges Teil, ein Handschuh oder eine tragbare Bandage ist.

11. Verwendung nach Anspruch 10, **dadurch gekennzeichnet, dass** das Mittel abnehmbar, austauschbar, waschbar oder desinfizierbar ist.

12. Verwendung nach Anspruch 10 oder 11, **dadurch gekennzeichnet, dass** das Mittel als Penis-Wundabdeckung oder Genital-Wundabdeckung dient.

13. Verwendung nach einem der Ansprüche 10 bis 12, **dadurch gekennzeichnet, dass** das Mittel bei Wunden, Operationswunden oder Behandlungsstellen der Haut im Genitalbereich, am Penis, am Skrotum, an Skrotum und Leiste, bei offenen Beinen, Mykosen, Dermatitis, Neurodermitis, Schuppenflechte, zum Schutz der Haut oder zur Prävention von Hautschädigungen oder Hautreizungen verwendet wird.

## Claims

1. Wound cover for a non-contact covering of a wound or skin area with at least one dimensionally stable cap (1) and a non-adhesive plane-like carrier (2), wherein said carrier (2) is a garment, a piece of undergarment, an underpant, a slip, a diaper, tights, compression tights, a stocking, a piece like a stocking, a glove or a wearable bandage and said cap (1) is arranged or fastened on top of the carrier (2).

2. Wound cover according to claim 1, wherein said cap (1) is assembled or fastened on said carrier (2) with its rim (3), which is located on the base surface.

3. Wound cover according to claim 1 or 2, wherein said cap (1) is connected to said carrier (2) in an inseparable, detachable, removable or interchangeable manner.

4. Wound cover according to claim 1 or 2, wherein said cap (1) is connected to said carrier (2) by sewing, glueing, welding, stapling, tacking, chemical or physical adhesion, mechanical action, hook-and-loop-tape, plug-in connection or snap into place connection .

5. Wound cover according to claims 1 to 4, wherein said cap (1) has one or more openings (5, 13, 14), one or more closable openings (5, 13, 14), one or more openings (5, 13, 14) with a passive or active valve, one or more slots or one or more channels (13, 14).

6. Wound cover according to claims 1 to 4, wherein said cap (1) has one or more inserts, one or more elements on top, one or more sealing elements in the rim area (3), one or more adhesive elements in the rim area (3), one or more adhesive elements or fastening means (4) on the inner surface or outer surface, one or more non-slip elements in the rim area (3) or one or more moisture or wetness absorbing elements or wetness binders.

7. Wound cover according to claims 1 to 4, wherein said cap (1) has one or more storage or dispensing devices for solid, liquid or gaseous drugs or therapeutically used substances or materials, one or more germ-killing substances or a drug-delivery device.

8. Wound cover according to claims 1 to 4, wherein said cap (1) has one or more windows or radiation transparent, radiation focussing or radiation filtering areas, one or more functional elements, a transparent, in part or partially transparent wall, an untransparent or for radiation impermeable wall, a means for ventilation or a gas inlet.

9. Wound cover according to claims 1 to 8, wherein said carrier (2) has an opening or recess (6) in the area on which said cap (1) is arranged or said carrier (2) has one or more non-slip elements on the body-facing side thereof.

10. Use of at least one dimensionally stable cap (1) with one or more fasteners (4) and a non-adhesive plane-like carrier (2) as a means for a contact-free coverage of a skin area or a non-contact wound covering, wherein said cap (1) is arranged or attached on said carrier (2) and said carrier (2) is a garment, a piece of undergarment, an underpant, a slip, a diaper, tights, compression tights, a stocking, a piece like a stocking, a glove or a wearable bandage.

11. Use according to claim 10, wherein said means is removable, replaceable, washable or can be disinfected.

12. Use according to claim 10 or 11, wherein said means serves as a penis-wound cover or genital wound cover.

13. Use according to claims 10 to 12, wherein said means is used for wounds, surgical wounds or places of treatment of the skin in the genital area, on the penis, on the scrotum, on scrotum and groin, leg ulcer, mycosis, dermatitis, neurodermitis, psoriasis, for protection of the skin or for the prevention of skin damage or skin irritation.

## Revendications

1. Couvre-plaie pour un couverture sans contact d'une plaie ou d'une zone de peau avec au moins un capuchon rigide (1) et un support (2) non adhésif et étendu, dans lequel le support (2) est un vêtement, une pièce de sous-vêtement, une paire de culottes, un feuillet, une couche-culotte, un collant, un collant de compression, un bas, une pièce comme un bas, un gant ou une bandage portable et le capuchon (1) est placé ou fixé sur le support (2).

2. Couvre-plaie selon la revendication 1, **caractérisé en ce que** le capuchon (1) est placé ou fixé sur le support (2) avec son bord (3), qui est situé à la base de capuchon.

3. Couvre-plaie selon la revendication 1 ou 2, **caractérisé en ce que** le capuchon (1)) est relié au support (2) d'une manière inséparable, détachable, amovible ou interchangeable.

4. Couvre-plaie selon la revendication 1 ou 2, **caractérisé en ce que** le capuchon (1)) est relié au support (2) par couture, collage, soudage, agrafage, fixage, force d'adhérence chimique ou physique, une action mécanique, velcro, branchement ou encliquetage.

5. Couvre-plaie selon une des revendications 1 à 4, **caractérisé en ce que** le capuchon (1) a une ou plusieurs ouvertures (5, 13, 14), une ou plusieurs ouvertures obturables (5, 13, 14), une ou plusieurs ouvertures (5, 13, 14) avec une soupape passive ou active, une ou plusieurs fentes ou un ou plusieurs canaux (13, 14).

6. Couvre-plaie selon une des revendications 1 à 4, **caractérisé en ce que** le capuchon (1) a un ou plusieurs insertions, un ou plusieurs éléments sur le dessus, un ou plusieurs éléments d'étanchéité dans la région de bord (3), un ou plusieurs éléments adhésifs dans la région de bord (3), un ou plusieurs éléments adhésifs ou moyens de fixation (4) sur la surface intérieure ou la surface extérieure, un ou plusieurs éléments anti-dérapants dans la région de bord (3) ou un ou plusieurs éléments d'absorption d'humidité ou éléments de fixage d'humidité.

7. Couvre-plaie selon une des revendications 1 à 4, **caractérisé en ce que** le capuchon (1) comprend un ou plusieurs dispositifs de stockage ou de distribution pour des médicaments solides, liquides ou gazeux ou pour des substances ou les matériaux thérapeutique, ou pour un ou plusieurs substances désinfectantes ou un dispositif de distribution de médicament.

8. Couvre-plaie selon une des revendications 1 à 4, **caractérisé en ce que** le capuchon (1) a une ou plusieurs fenêtres ou zones transparente pour radiation ou zones de focalisation ou de filtrage de radiation, ou un ou plusieurs éléments fonctionnels, un paroi transparente, un paroi en partie ou partiellement transparente, un paroi non-transparente, un paroi imperméable pour radiation, un moyen pour la ventilation ou une entrée de gaz.

9. Couvre-plaie selon une des revendications 1 à 8, **caractérisé en ce que** le support (2) comprend une ouverture ou évidement dans la la région à laquelle le capuchon (1) est disposé ou le support (2) a un ou plusieurs éléments anti-dérapants sur le côté faisant face au corps.

10. Utilisation d'au moins un capuchon rigide (1) avec un ou plusieurs moyens de fixation (4) et un support (2) non adhésif et étendu, en tant que moyen pour une couverture d'une zone de peau ou couvre-plaie sans contact,
dans lequel le capuchon (1) est placé ou fixé sur le support (2) et
le support (2) est un vêtement, une pièce de sous-vêtement, une paire de culottes, un feuillet, une couche-culotte, un collant, un collant de compression, un bas, une pièce comme un bas, un gant ou une bandage portable.

11. Utilisation selon la revendication 10, **caractérisé en ce que** le moyen est amovibles, remplaçable, lavable ou désinfectable.

12. Utilisation selon la revendication 10 ou 11, **caractérisé en ce que** le moyen est en tant que couverture du pénis-plaie ou des plaies génitales.

13. Utilisation selon une des revendications 10 à 12, **caractérisé en ce que** le moyen est pour les plaies, les plaies chirurgicales ou des points de traitement de la peau dans la région génitale, le pénis, le scrotum, le scrotum et l'aine, ulcère jambier, les mycoses, les dermatites, l'eczéma, le psoriasis, à la protection de la peau ou pour la prévention des dommages à la peau ou des irritations de la peau.
